# EUROPEAN PATENT APPLICATION

(11) **EP 4 732 745 A1**
(43) Date of publication of application: **29.04.2026**
(21) Application number: 24208487.9
(22) Date of filing: 23.10.2024
(51) Int. Cl.: A61B 1/00

(54) **A MEDICAL VIEWING SYSTEM WITH A VIEWING SCOPE, AN ENCAPSULATION FOR THE VIEWING SCOPE AND A METHOD OF OPERATING THE VIEWING SYSTEM**

(71) Applicant: LiNA Medical International Operations AG, 6039 Root D4 (CH)
(72) Inventor: Bjørn-Rasmussen, Peter, 6039 Root D4 (CH)
(74) Representative: Inspicos P/S

(57) **Abstract**

A medical viewing system comprising a viewing scope and an encapsulation for the viewing scope, where the encapsulation defines a conduit formed by a tubular element and a locking piece. To allow close contact between the viewing scope and a transparent viewing cap through which the viewing scope captures the imaging, the tubular element can move between an unexpanded position to an expanded position relative to the locking piece.

## Description

### INTRODUCTION

The invention relates to a medical viewing system comprising a viewing scope and an encapsulation for encapsulating at least a distal, insertable, part of the viewing scope.

The viewing scope comprises an elongated insertion part with an image capturing distal end.

The encapsulation defines an encapsulation conduit extending axially between a proximal end and a distal end, the proximal end forming a proximal opening for receiving the elongated insertion part in the encapsulation conduit and the distal end defining a transparent viewing cap terminating and concealing the encapsulation conduit.

The invention further relates to the encapsulation, and to a method of using the viewing system.

### BACKGROUND

In various medical procedures, inter alia gynaecological or laparoscopic procedures, a scope is inserted into a body cavity. The scope enables visualization of patient tissue and typically allows simultaneous treatment or taking tissue samples. For this reason, the scopes may include a channel for a medical instrument inserted through a passage of the scope. Moreover, water or other liquids may be applied for distending the body cavity or for cleaning the lens of the scope.

After the procedure, the scope is cleaned and sterilized.

Typically, the cleaning and sterilisation is expensive and time consuming.

### SUMMARY

It is objects of embodiments of this disclosure to allow a more efficient and less costly use of medical viewing scopes. Particularly, it is an object to avoid contamination and to avoid sterilisation. It is a further object to enable a more efficient medical procedure and to improve the quality of the imaging.

According to these and other objects, the disclosure, in a first aspect, provides a medical viewing system according to claim 1.

Since the tubular element can move from an unexpanded position to an expanded position relative to the locking piece, it can be ensured that the image capturing end is completely flush against the viewing cap. Particularly, this feature may prevent an air gap between the viewing cap and the image capturing end. This may improve the image quality and provide an easy and efficient way of ensuring correct positioning of the viewing scope in the encapsulation.

Accordingly, the invention improves encapsulation of scopes for medical procedures and enables increased sterility and avoidance of sterilisation of used scopes while still offering a good imaging quality.

The medical viewing system could be a hysteroscope system for gynaecological examination, it could be a resectoscope system for inspection of urethra and or the urinary tract, or it could be other scope systems for inspection of internal body parts.

The viewing scope could be a commercially available viewing scope, e.g., of the kind sold under the brand name Versascope^{™}, ALPHASCOPE^{™}, or other scopes.

Such viewing scopes known in the art normally forms an elongated, tubular, insertion part with an image capturing distal end. The distal end is either formed with a camera or by a lens system transmitting the image to the opposite, proximal end.

The scope is typically made by durable and expensive materials allowing autoclave and extensive reuse. For easy handling, the scope can be isolated from the patient by the encapsulation. The encapsulation, on the contrary, could be made of low-cost materials for single-use purpose, including plastic materials known for one time use in medical procedures.

Accordingly, the parts coming in direct contact with the patient can be discharged after the surgery while the expensive scope can be reused, optimally without sterilisation, depending on the procedure in question.

The encapsulation conduit could be formed by a tubular element such as a steel element. Optionally, it could be formed in two parts which are assembled e.g., adhesively. It could e.g., be made of a steel tube forming the distal end and a plastic element forming the proximal end.

The locking piece could be made from a plastic material and could particularly form a handpiece for handling the encapsulation.

The locking piece comprises the scope lock which is configured to releasably lock the viewing scope. Subsequently, the scope and the encapsulation can be handled as a single entity without axial sliding of the scope relative to the encapsulation. The scope lock may form a projection or a depression arranged for interlocking engagement with the viewing scope. The scope lock may be located in the encapsulation conduit.

The viewing scope may comprise a locking feature configured for engagement with the scope lock. The locking feature and the scope lock could be geometrically matching features of the encapsulation and the scope which interlock when the scope is inserted in the encapsulation conduit. This may provide an effective and simple interlocking and may further ensure that the scope and the encapsulation works as a match thereby preventing use of a wrong scope or wrong encapsulation for a specific procedure. The locking feature may e.g. comprise a projecting element being received behind a projecting element forming the scope lock of the locking piece or being received in a depression forming the scope lock of the locking piece, or in any other similar manner interlocking with the scope lock of the locking piece. Alternatively, the locking feature may e.g. comprise a depression receiving a projecting element forming the scope lock of the locking piece or in any other similar manner interlocking with the scope lock of the locking piece.

A distance from the locking feature to the image capturing may exceed a distance from the scope lock to the distal end in the unexpanded position. By this feature it is necessary for the engagement between the scope lock and the locking feature that the tubular element moves to the expanded position and thus ensures close contact between the image capturing end and the viewing cap. In one embodiment, the exceeding distance may be in the range of 2-10 millimetres such as 3-8 millimetres.

The scope lock may be configured to releasably lock the viewing scope at a plurality of positions of the viewing scope in the encapsulation conduit. That may allow a more flexible positioning of the viewing scope, and it may enable use of viewing scopes and/or encapsulations having different length.

In use, the practitioner will simply move the scope until the tubular element displaces relative to the locking piece and, in this process, the scope lock may lock the position of the viewing scope at a suitable position. Due to the displacement of the tubular element, the close contact between the image capturing end of the viewing scope and the transparent viewing cap is ensured.

The plurality of positions may be established by the scope and/or the locking piece having a high friction surface preventing sliding in one direction and allowing sliding in an opposite direction, e.g. a jagged or serrated surface.

The joint may comprise an elastically deformable member providing an elastic deformation force when moving the tubular element from the unexpanded position to the expanded position. The elastically deformable member could be a helical spring, other kinds of springs, a piece of a foam material or rubber material or another kind of material having an elastic compressibility.

The joint may comprise a plastic deformable member providing a deformation force when moving the tubular element from the unexpanded position to the expanded position, and wherein the image capturing end engages the viewing cap and urges the tubular element towards the expanded position against the deformation force.

The locking piece may comprise an inner tubular wall encircling a locking section of the encapsulation conduit. The tubular element may comprise an outer tubular wall encircling the locking section of the encapsulation conduit, and the inner wall may have an inner surface towards the encapsulation conduit and an outer surface towards the outer tubular wall. In this setup, the outer tubular wall may have an inner surface sliding against the outer surface of the inner tubular wall during the displacement from the unexpanded position to the expanded position. That may provide good stability and reduce kinking of the encapsulation conduit upon movement to the expanded position.

The locking piece may comprise an external tubular wall encircling the locking section of the encapsulation conduit and may comprise an inner surface towards the outer tubular wall and an outer surface forming a handpiece for gripping the encapsulation.

The inner surface of the inner tubular wall towards the encapsulation conduit may define the scope lock, e.g. by defining a projection or a depression arranged to engage a part of the elongated insertion part of the viewing scope.

The elastically deformable member may be arranged in a space between the external tubular wall and outer tubular wall, e.g. limited in the distal end of the space by the tubular element and limited in proximal end of the space by the locking piece.

Due to the space defined between the external tubular wall and the outer tubular wall, important features of the encapsulation, mainly the scope lock and the elastically or plastically deformable member can be housed at or around the locking section of the encapsulation conduit. Accordingly, the part of the locking piece at the locking section may have an increased diameter compared to the remaining part of the encapsulation. This enables the section to be used as a handpiece for gripping and manipulating the encapsulation.

The tubular element may define a first section of the encapsulation conduit towards the distal end. This first section of the encapsulation conduit may have a uniform cross section.

The tubular element may define a second section of the encapsulation conduit towards the proximal end, and the second section of the encapsulation conduit may have a non-uniform cross section.

The viewing scope may comprise a handle part from which the elongated insertion part projects. The encapsulation may comprise a pouch connected to the locking piece and configured for encapsulation of the handle part. The pouch may completely prevent contamination of the scope and therefore further facilitate reuse of the scope, e.g., without sterilisation depending on the procedure in question.

The pouch could be connected to the locking piece in a releasable connection structure, e.g. comprising an elastically deformable ring circumferentially enclosing and pressing a part of the pouch against an outer surface of the locking piece. This may allow easy detachment of the pouch and even enable the pouch to be reattached to the locking piece. It may facilitate sorting of the parts for improved waste handling.

The handpiece formed by the locking piece may be encapsulated by the pouch together with the viewing scope.

The encapsulation may further define a flushing system for flushing liquids into or out of the encapsulation conduit. The flushing system may comprise a flushing conduit extending from an open end allowing intake of a flushing liquid to a flushing inlet in the tubular element. Since the inlet is in the tubular element as opposed to being in the locking piece, the flushing system follows the movement of the tubular element when it is displaced to the expanded position. This allows flushing in the expanded position or even during movement between the unexpanded and the expanded positions.

The encapsulation may further comprise a working channel, e.g., an expandable working channel. The working channel may be separated from the tubular element and the locking piece to thereby become unaffected by displacement of the tubular element from the unexpanded position to the expanded position.

The working channel may extend in parallel with the encapsulation conduit between a tool inlet in a proximal end of the working channel and a tool outlet in a distal end of the working channel. The tool inlet may comprise a duckbill valve for sealed insertion of a tool, and the tool outlet may be located either at a distance from the transparent viewing cap or close to the transparent viewing cap, e.g., right next to the transparent viewing cap.

The tools could include various tools known in the art, e.g., including forceps etc.

In a second aspect, the disclosure provides an encapsulation for a viewing scope. The encapsulation being like the encapsulation of the medical viewing system according to the first aspect and may include any of the features mentioned in relation to the first aspect.

In a third aspect, the disclosure provides an encapsulation for a viewing scope defining an elongated insertion part terminating in an image capturing end, the encapsulation defining an encapsulation conduit extending axially between a proximal end and a distal end, the proximal end forming a proximal opening for receiving the elongated insertion part in the encapsulation conduit and the distal end defining a transparent viewing cap terminating and concealing the encapsulation conduit.

The encapsulation conduit is formed by a tubular element and a locking piece, where the tubular end forms the distal end and the locking piece forms the proximal end.

The locking piece comprises a scope lock configured to releasably lock the viewing scope at a first predetermined position of the viewing scope in the encapsulation conduit and, when locked, configured to prevent axial displacement of the scope relative to the locking piece, and the encapsulation comprises a pouch connected to the locking piece and configured for encapsulation of the handle part.

The pouch could be connected to the locking piece in a releasable connection structure, e.g., formed by an elastically deformable ring circumferentially enclosing and pressing a part of the pouch against an outer surface of the locking piece.

The tubular element and the locking piece could be connected by a joint configured for allowing displacement of the tubular element against a deformation force from an unexpanded position to an expanded position relative to the locking piece thereby increasing a distance between the proximal end and the distal end.

In a fourth aspect, the disclosure provides a method of using a viewing system according to the first aspect. The method comprises:
- inserting the elongated insertion part into the encapsulation conduit until the image capturing end abuts the viewing cap; and
- holding the locking piece while inserting the elongated insertion part further into the encapsulation conduit until the scope lock locks the viewing scope while deforming the joint and while displacing the tubular element from the unexpanded position to the expanded position.

The viewing system could be used while it is concealed by the encapsulation conduit and the pouch.

The method may comprise detaching the pouch from the locking piece after use of the viewing scope.

A flushing liquid may be flushed into the flushing conduit while the tubular element is in the expanded position, and a tool may be inserted into the working channel while the tubular element is in the expanded position.

### BRIEF DESCRIPTION OF THE DRAWING

Fig. 1 illustrates a medical viewing system;
Fig. 2 illustrates the tubular element and the locking piece in an exploded view;
Fig. 3 illustrates the locking piece with the scope lock;
Fig. 4 illustrates the scope with a locking feature;
Fig. 5 illustrates a distal end of the tubular element;
Figs. 6 and 7 illustrate movement from the unexpanded to the expanded configuration;
Fig. 8 illustrates a multiple position locking feature;
Figs. 9 and 10 illustrate exploded views of the tubular element and the locking piece;
Fig. 11 illustrates a proximal end of the locking piece;
Fig. 12 illustrates the encapsulation comprising a pouch;
Figs. 13 and 14 illustrate details of the attachment of the pouch to the locking piece; and
Figs. 15 and 16 illustrate an embodiment of the encapsulation including a working channel and a flushing system.

### DETAILED DESCRIPTION

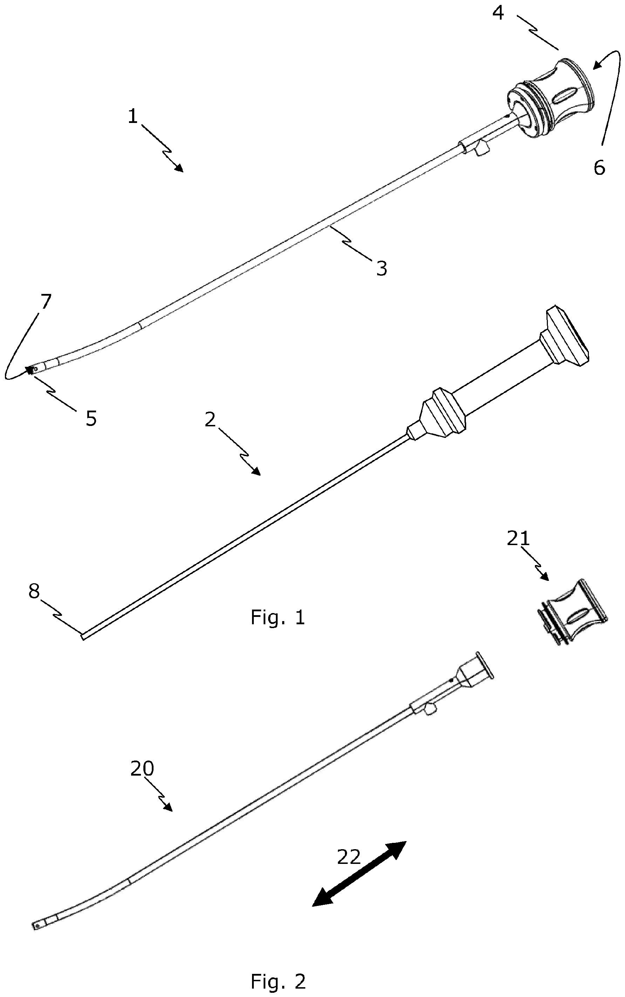

Fig. 1 illustrates a medical viewing system comprising an encapsulation 1 for a medical viewing scope 2. The encapsulation is for encapsulating a viewing scope during a medical procedure, e.g., a hysteroscopic procedure.

The encapsulation defines an encapsulation conduit 3 extending axially between a proximal end 4 and a distal end 5. Herein, proximal end refers to the end facing the practitioner, and the distal end is the end facing the patient during use.

The proximal end forms a proximal opening 6 for receiving an elongated insertion part of the viewing scope, and the distal end defines a transparent viewing cap 7 terminating and concealing the encapsulation conduit. The viewing cap forms a window allowing the image capturing end 8 of the viewing scope 2 to capture images through the viewing cap while still isolating the viewing scope 2 from the patient.

Fig. 2 is an exploded view illustrating the two distinct elements forming the encapsulation conduit. The two distinct elements are referred to herein as a tubular element 20 and a locking piece 21. The tubular element forms the distal end with the viewing cap, and the locking piece forms the proximal end with the proximal opening 6.

The tubular element and the locking piece are connected by a joint allowing displacement, illustrated by the arrow 22, of the tubular element from an unexpanded position to an expanded position relative to the locking piece thereby increasing a distance between the proximal end and the distal end.

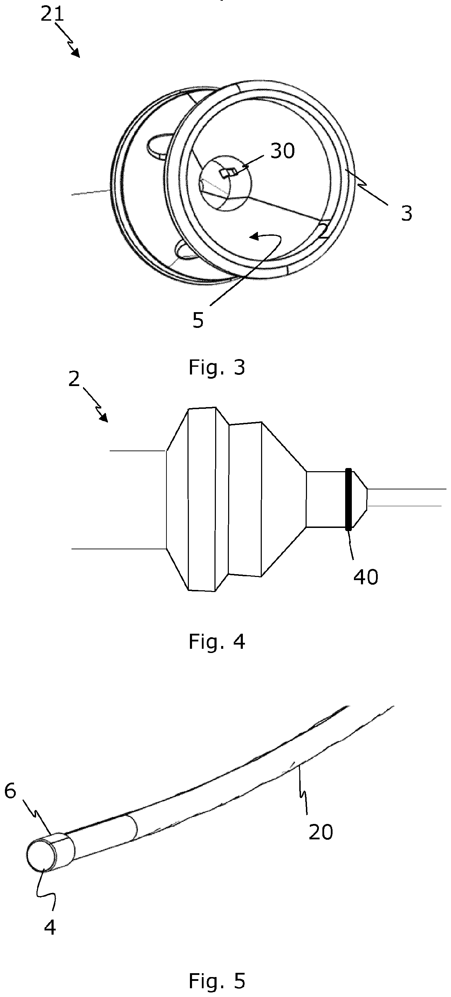

Fig. 3 illustrates the locking piece seen from the proximal end. The locking piece forms a projection, projecting into the encapsulation conduit. The projection constitutes a scope lock 30 which releasably engages and locks the viewing scope when reaching a specific position in the encapsulation conduit. Herein, this position is referred to as *a first predetermined position.*

Once the scope lock engages the viewing scope, the locked configured prevents axial displacement of the scope out of the encapsulation conduit.

Fig. 4 illustrates a handle part of the viewing scope 2. The handle part has a geometric feature in the form of a projecting collar 40 which, when pressed behind the scope lock prevents axial movement of viewing scope relative to the encapsulation.

Fig. 5 illustrates a part of the tubular element 20 extending towards the distal end 4. Fig. 4 also illustrates the viewing cap 6 which seals the distal end of the encapsulation conduit.

Before initiating medical procedure, the practitioner inserts the elongated insertion part of the viewing scope into the encapsulation conduit until the image capturing end thereof abuts the viewing cap. Subsequently, the viewing scope is pushed further into the encapsulation conduit until the scope lock locks the axial position of the viewing scope. In this position, the viewing scope has pushed the tubular element 20 towards the expanded configuration. This ensures complete contact between the image capturing end and the viewing cap and therefore improves the image quality when using an encapsulated viewing scope by reducing or preventing a gap between the viewing cap and the image capturing end.

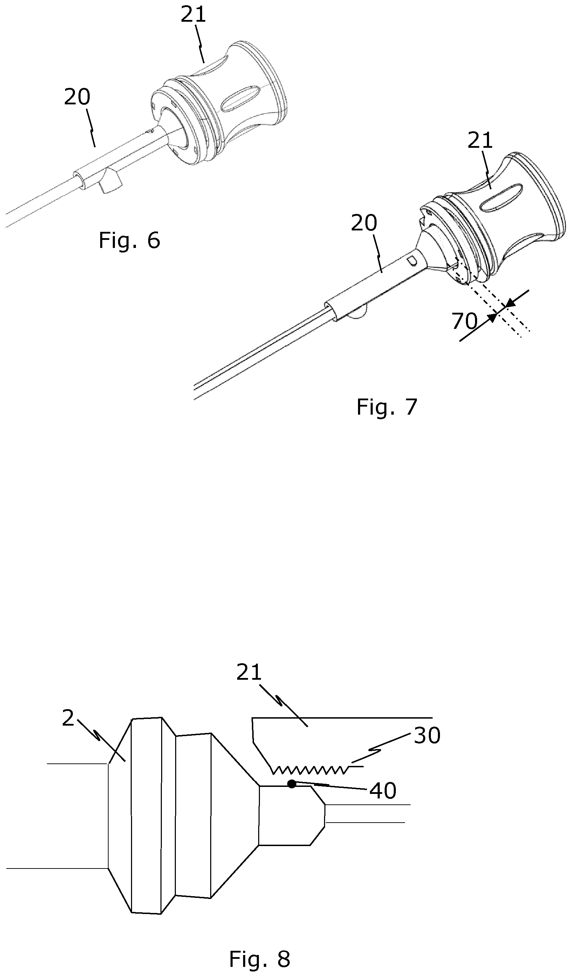

Fig. 5 illustrates the distal end of the tubular element 20 in the unexpanded position relative to the locking piece 21. Fig. 6 illustrates the distal end of the tubular element 20 in the expanded position relative to the locking piece 21 and indicates the elongation by the arrow 70.

Fig. 8 illustrates schematically an embodiment of the scope lock 30 configured to releasably lock the viewing scope at a plurality of positions of the viewing scope in the encapsulation conduit. In this schematic illustration, the geometric feature 40 of the viewing scope 2 is illustrate as a projection which slides on the toothed structure constituting the scope lock 30.

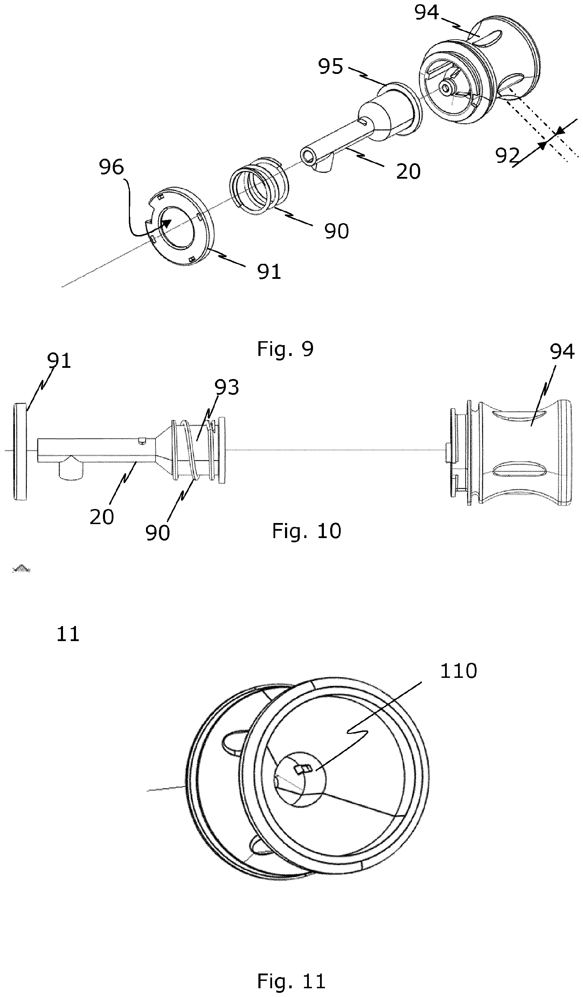

Figs. 9-11 illustrate an embodiment where the joint comprises an elastically deformable member providing an elastic deformation force when moving the tubular element from the unexpanded position to the expanded position. In the illustrated embodiment, this elastically deformable member is constituted by a helical spring 90. In alternative embodiments, it could be a piece of a deformable material such as a foam material or rubber etc.

Fig. 9 illustrates the locking piece 21, the proximal end of the tubular element 20, the spring 90, and a locking washer 91, seen from the distal end in an exploded, perspective view. Fig. 10 illustrates the locking piece, the proximal end of the tubular element, the spring, and the locking washer seen in a side view in which the spring is placed in its intended position around the proximal end of the tubular element. Fig. 11 illustrates the locking piece seen from the proximal end.

In the embodiment disclosed in Fig. 9, the locking piece comprises an inner tubular wall encircling a locking section of the encapsulation conduit, wherein the tubular element comprises an outer tubular wall encircling the locking section of the encapsulation conduit, wherein the inner wall has an inner surface towards the encapsulation conduit and an outer surface towards the outer tubular wall, and wherein the outer tubular wall has an inner surface sliding against the outer surface of the inner tubular wall during the displacement from the unexpanded position to the expanded position.

The locking piece defines a locking section 92 encompassing the scope lock 30. The external tubular wall 110 encircles the locking section 92 of the encapsulation conduit and comprises an inner surface towards the encapsulation conduit and defining the scope lock.

The tubular element 20 comprises an outer tubular wall 93 encircling the locking section of the encapsulation conduit.

The inner wall of the external tubular wall 110 has an outer surface towards the outer tubular wall and the outer tubular wall has an inner surface sliding against the outer surface of the inner tubular wall during the displacement from the unexpanded position to the expanded position. This provides a good stability of the encapsulation during movement to the expanded position.

The locking piece defines an external tubular wall 94 encircling the locking section of the encapsulation conduit and comprises an inner surface towards the outer tubular wall and an outer surface forming a handpiece for gripping the encapsulation.

The elastically deformable member 90, in this case a helical spring, is arranged in a space between the external tubular wall and outer tubular wall which means that the spring is directly within the handpiece for gripping the encapsulation. That provides a good balance during movement to the expanded position against the force from the spring.

When the tubular element 20 is moved to the expanded position, the spring is compressed between the flange 95 of the tubular element 20 and the locking washer 91 which is attached to the locking piece 21. Accordingly, the tubular element 20 is allowed to slide back and forth through the hole 96 in the locking washer 91 under influence of the elastically deformable member 90.

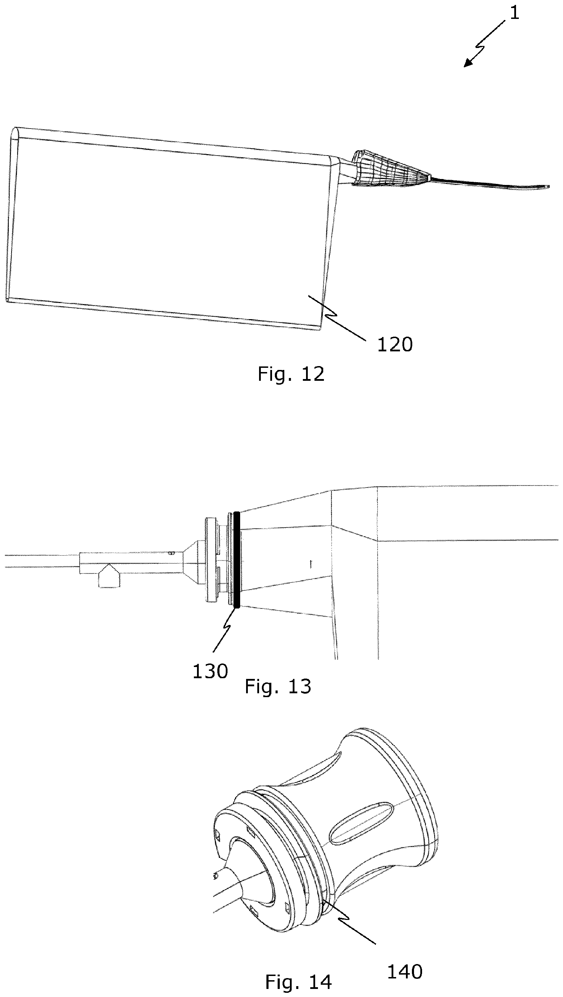

Fig. 12 illustrates an embodiment of the encapsulation 1 comprising a pouch 120 connected to the locking piece 21 and configured for encapsulation of the handle part of the viewing scope 2.

Fig. 13 illustrates an enlarged view of the pouch 120 and a rubber band 130 fixing the pouch to the handpiece formed by the locking piece 21. The rubber band 130 forms a deformable ring circumferentially enclosing and pressing a part of the pouch 120 against an outer surface of the locking piece. For fixing the rubber band 130 and thus the pouch 120 to the locking piece 21, the locking piece comprises a groove 140 illustrated in Fig. 14.

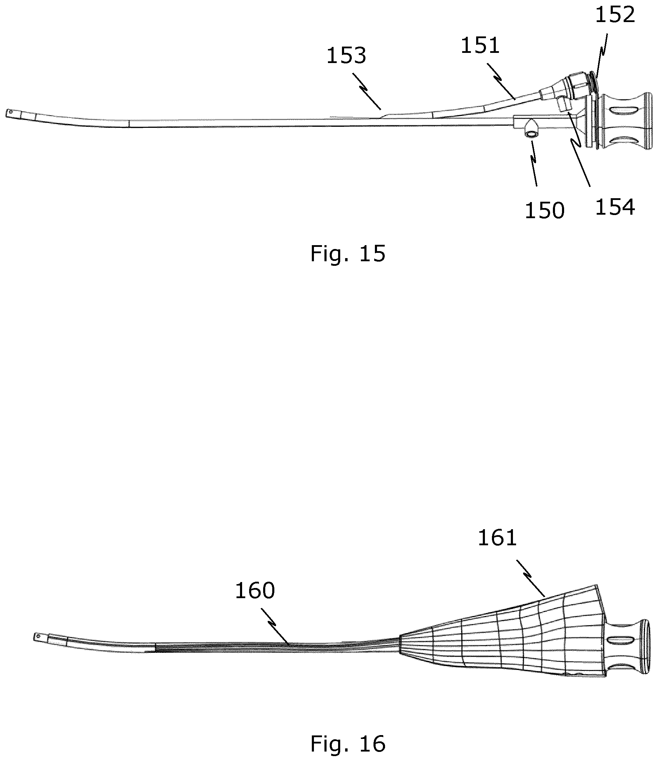

Figs. 15 and 16 illustrate an embodiment of the encapsulation including a working channel and a flushing system.

Fig. 15 illustrates a flushing system for flushing liquids into or out of the encapsulation conduit. The flushing system comprises a flushing conduit extending from an open end 150 allowing intake of a flushing liquid and ends inside the tubular element. This has the advantage of allowing continued flushing while moving the tubular element, even while moving the tubular element relative to the locking piece.

The encapsulation further comprises a working channel 151 extending along the encapsulation conduit between a tool inlet 152 in the proximal end of the working channel and a tool outlet 153 in the distal end of the working channel.

The working channel is separated from the tubular element and the locking piece, i.e. it constitutes a separate component, and it is therefore not affected by displacement of the tubular element from the unexpanded position to the expanded position. Accordingly, expansion of the encapsulation conduit does not affect the working channel. In use, the working channel may allow insertion of e.g., a forceps, a surgical snare, or any other similar tools known for minimal invasive surgery.

The working channel forms a working channel flushing conduit extending from an open end 154 allowing intake of a flushing liquid and ends inside the working channel.

Fig. 16 illustrates the encapsulation with a wrap of an elastic hose 160 covering the tubular element 20 and the working channel 151. The hose is connected to a housing 161 covering the locking piece 21. The elastic characteristic of the hose allows deformation of the hose if a tool is inserted into the working channel.

### LIST OF NUMBERED EMBODIMENTS

1. A medical viewing system comprising a viewing scope and an encapsulation for the viewing scope, the viewing scope comprises an elongated insertion part with an image capturing end, and the encapsulation defining an encapsulation conduit extending axially between a proximal end and a distal end, the proximal end forming a proximal opening for receiving the elongated insertion part in the encapsulation conduit and the distal end defining a transparent viewing cap terminating and concealing the encapsulation conduit;
   wherein the encapsulation conduit is formed by a tubular element and a locking piece, where the tubular element forms the distal end and the locking piece forms the proximal end,
   wherein the tubular element and the locking piece are connected by a joint allowing displacement of the tubular element from an unexpanded position to an expanded position relative to the locking piece thereby increasing a distance between the proximal end and the distal end,
   wherein the locking piece comprises a scope lock configured to releasably lock the viewing scope at a first predetermined position of the viewing scope in the encapsulation conduit and, when locked, configured to prevent axial displacement of the scope out of the encapsulation conduit, and
   wherein the image capturing end, in the first predetermined position of the viewing scope engages the viewing cap and urges the tubular element towards the expanded position.
2. The medical viewing system according to embodiment 1, wherein the viewing scope comprises a locking feature configured for engagement with the scope lock, and wherein the engagement occurs at a position of the viewing scope relative to the encapsulation conduit where the image capturing end engages the viewing cap.
3. The medical viewing system according to embodiment 2, wherein a distance from the locking feature to the image capturing end exceeds a distance from the scope lock to the distal end in the unexpanded position such that engagement between the scope lock and the locking feature necessitates displacement of the tubular element to the expanded position.
4. The medical viewing system according to any of the preceding embodiments, wherein the scope lock is configured to releasably lock the viewing scope at a plurality of positions of the viewing scope in the encapsulation conduit and, when locked, configured to prevent axial displacement of the viewing scope relative to the locking piece out of the encapsulation conduit.
5. The medical viewing system according to any of the preceding embodiments, wherein the joint comprises an elastically deformable member providing an elastic deformation force when moving the tubular element from the unexpanded position to the expanded position, and wherein the image capturing end engages the viewing cap and urges the tubular element towards the expanded position against the deformation force.
6. The medical viewing system according to any of the preceding embodiments, wherein the joint comprises a plastic deformable member providing a deformation force when moving the tubular element from the unexpanded position to the expanded position, and wherein the image capturing end engages the viewing cap and urges the tubular element towards the expanded position against the deformation force.
7. The medical viewing system according to any of the preceding embodiments, wherein the locking piece comprises an inner tubular wall encircling a locking section of the encapsulation conduit, wherein the tubular element comprises an outer tubular wall (93) encircling the locking section of the encapsulation conduit, wherein the inner wall has an inner surface towards the encapsulation conduit and an outer surface towards the outer tubular wall (93), and wherein the outer tubular wall (93) has an inner surface sliding against the outer surface of the inner tubular wall during the displacement from the unexpanded position to the expanded position.
8. The medical viewing system according to embodiment 7, wherein the locking piece comprises an external tubular wall encircling the locking section of the encapsulation conduit and comprises an inner surface towards the outer tubular wall (93) and an outer surface forming a handpiece for gripping the encapsulation.
9. The medical viewing system according to embodiments 7 or 8, wherein the inner surface of the inner tubular wall towards the encapsulation conduit defines the scope lock.
10. The medical viewing system according to embodiment 5 and any of the embodiments 7-9, wherein the elastically deformable member is arranged in a space between the external tubular wall and outer tubular wall (93).
11. The medical viewing system according to embodiment 10, wherein the spring is limited in the distal end of the space by the tubular element and limited in proximal end of the space by the locking piece.
12. The medical viewing system according to any of the preceding embodiments, wherein the tubular element defines a first section of the encapsulation conduit towards the distal end, wherein the first section of the encapsulation conduit has a uniform cross section.
13. The medical viewing system according to any of the preceding embodiments, wherein the tubular element defines a second section of the encapsulation conduit towards the proximal end, wherein the second section of the encapsulation conduit has a non-uniform cross section.
14. The medical viewing system according to any of the preceding embodiments, wherein the viewing scope comprises a handle part from which the elongated insertion part projects, and wherein the encapsulation comprises a pouch connected to the locking piece and configured for encapsulation of the handle part.
15. The medical viewing system according to embodiment 14, wherein the pouch is connected to the locking piece in a releasable connection structure.
16. The medical viewing system according to embodiment 15, wherein the releasable connection structure comprises an elastically deformable ring circumferentially enclosing and pressing a part of the pouch against an outer surface of the locking piece.
17. The medical viewing system according to embodiments 8 and 16, wherein the handpiece is encapsulated by the pouch.
18. The medical viewing system according to any of the preceding embodiments, further comprising a flushing system for flushing liquids into or out of the encapsulation conduit, the flushing system comprising a flushing conduit extending from an open end allowing intake of a flushing liquid to a flushing inlet in the tubular element.
19. The medical viewing system according to any of the preceding embodiments, further comprising a working channel extending along the encapsulation conduit between a tool inlet in a proximal end of the working channel and a tool outlet in a distal end of the working channel, the working channel being separated from the tubular element and the locking piece to thereby become unaffected by displacement of the tubular element from the unexpanded position to the expanded position.
20. An encapsulation for a viewing scope defining an elongated insertion part terminating in an image capturing end, the encapsulation defining an encapsulation conduit extending axially between a proximal end and a distal end, the proximal end forming a proximal opening for receiving the elongated insertion part in the encapsulation conduit and the distal end defining a transparent viewing cap terminating and concealing the encapsulation conduit;
   wherein the encapsulation conduit is formed by a tubular element and a locking piece, where the tubular element forms the distal end and the locking piece forms the proximal end,
   wherein the tubular element and the locking piece are connected by a joint allowing displacement of the tubular element from an unexpanded position to an expanded position relative to the locking piece thereby increasing a distance between the proximal end and the distal end,
   wherein the locking piece comprises a scope lock configured to releasably lock the viewing scope at a first predetermined position of the viewing scope in the encapsulation conduit and, when locked, configured to prevent axial displacement of the scope out of the encapsulation conduit.
21. The encapsulation according to embodiment 20, wherein the scope lock is configured to releasably lock the viewing scope at a plurality of positions of the viewing scope in the encapsulation conduit and, when locked, configured to prevent axial displacement of the scope relative to the locking piece out of the encapsulation conduit.
22. The encapsulation according to any of embodiments 20-21, wherein the joint comprises an elastically deformable member providing an elastic deformation force when moving the tubular element from the unexpanded position to the expanded position.
23. The encapsulation according to any of embodiments 20-22, wherein the joint comprises a plastic deformable member providing an elastic deformation force when moving the tubular element from the unexpanded position to the expanded position.
24. The encapsulation according to any of embodiments 20-23, further comprising a flushing system for flushing liquids into or out of the encapsulation conduit, the flushing system comprising a flushing conduit extending from an open end allowing intake of a flushing liquid to a flushing inlet in the tubular element.
25. The encapsulation according to any of embodiments 20-24, further comprising a working channel extending along the encapsulation conduit between a tool inlet in a proximal end of the working channel and a tool outlet in a distal end of the working channel, the working channel being separated from the tubular element and the locking piece to thereby become unaffected by displacement of the tubular element from the unexpanded position to the expanded position.
26. An encapsulation for a viewing scope defining an elongated insertion part terminating in an image capturing end, the encapsulation defining an encapsulation conduit extending axially between a proximal end and a distal end, the proximal end forming a proximal opening for receiving the elongated insertion part in the encapsulation conduit and the distal end defining a transparent viewing cap terminating and concealing the encapsulation conduit;
   wherein the encapsulation conduit is formed by a tubular element and a locking piece, where the tubular element forms the distal end and the locking piece forms the proximal end,
   wherein the locking piece comprises a scope lock configured to releasably lock the viewing scope at a first predetermined position of the viewing scope in the encapsulation conduit and, when locked, configured to prevent axial displacement of the scope relative to the locking piece; and
   wherein the encapsulation comprises a pouch connected to the locking piece and configured for encapsulation of the handle part.
27. The encapsulation according to embodiment 26, wherein the pouch is connected to the locking piece in a releasable connection structure.
28. The encapsulation according to embodiment 27, wherein the releasable connection structure comprises an elastically deformable ring circumferentially enclosing and pressing a part of the pouch against an outer surface of the locking piece.
29. The encapsulation according to embodiments 26-28, wherein the tubular element and the locking piece are connected by a joint configured for allowing displacement of the tubular element against a deformation force from an unexpanded position to an expanded position relative to the locking piece thereby increasing a distance between the proximal end and the distal end.
30. The encapsulation according to any of embodiments 26-29, further comprising a flushing system for flushing liquids into or out of the encapsulation conduit, the flushing system comprising a flushing conduit extending from an open end allowing intake of a flushing liquid to a flushing inlet in the tubular element.
31. The encapsulation according to any of embodiments 29-30, further comprising a working channel extending along the encapsulation conduit between a tool inlet in a proximal end of the working channel and a tool outlet in a distal end of the working channel, the working channel being separated from the tubular element and the locking piece to thereby become unaffected by displacement of the tubular element from the unexpanded position to the expanded position.
32. A method of using a viewing system according to any of embodiments 1-19, the method comprising:
   - inserting the elongated insertion part into the encapsulation conduit until the image capturing end abuts the viewing cap; and
   - holding the locking piece while inserting the elongated insertion part further into the encapsulation conduit until the scope lock locks the viewing scope while deforming the joint and displacing the tubular element from the unexpanded position to the expanded position.
33. The method according to embodiment 32, wherein the viewing system is in accordance with embodiment 14, and the viewing scope is used while it is concealed by the encapsulation conduit and the pouch.
34. The method according to embodiment 33, comprising detaching the pouch from the locking piece after use of the viewing scope.
35. The method according to any of embodiments 32-34, wherein the viewing system is in accordance with embodiment 18, and wherein a flushing liquid is flushed into the flushing conduit while the tubular element is in the expanded position.
36. The method according to any of embodiments 32-35, wherein the viewing system is in accordance with embodiment 19, and wherein a tool is inserted into the working channel while the tubular element is in the expanded position.

## Claims

1. A medical viewing system comprising a viewing scope (2) and an encapsulation (1) for the viewing scope (2), the viewing scope (2) comprises an elongated insertion part with an image capturing end (8), and the encapsulation defining an encapsulation conduit (3) extending axially between a proximal end (4) and a distal end (5), the proximal end forming a proximal opening (6) for receiving the elongated insertion part in the encapsulation conduit and the distal end defining a transparent viewing cap (7) terminating and concealing the encapsulation conduit;
wherein the encapsulation conduit (3) is formed by a tubular element (20) and a locking piece (21), where the tubular element (20) forms the distal end and the locking piece (21) forms the proximal end,
wherein the tubular element (20) and the locking piece (21) are connected by a joint allowing displacement of the tubular element (20) from an unexpanded position to an expanded position relative to the locking piece (21) thereby increasing a distance between the proximal end (4) and the distal end (5),
wherein the locking piece (21) comprises a scope lock (30) configured to releasably lock the viewing scope (2) at a first predetermined position of the viewing scope in the encapsulation conduit (3) and, when locked, configured to prevent axial displacement of the scope (2) out of the encapsulation conduit (3), and
wherein the image capturing end (8), in the first predetermined position of the viewing scope engages the viewing cap (7) and urges the tubular element (20) towards the expanded position.

2. The medical viewing system according to claim 1, wherein the viewing scope (2) comprises a locking feature (40) configured for engagement with the scope lock (30), and wherein the engagement occurs at a position of the viewing scope (2) relative to the encapsulation conduit (3) where the image capturing end (8) engages the viewing cap (7).

3. The medical viewing system according to claim 2, wherein a distance from the locking feature (40) to the image capturing end (8) exceeds a distance from the scope lock (30) to the distal end (5) in the unexpanded position such that engagement between the scope lock (30) and the locking feature (40) necessitates displacement of the tubular element (20) to the expanded position.

4. The medical viewing system according to any of the preceding claims, wherein the scope lock (30) is configured to releasably lock the viewing scope (2) at a plurality of positions of the viewing scope (2) in the encapsulation conduit (3) and, when locked, configured to prevent axial displacement of the viewing scope (2) relative to the locking piece (21) out of the encapsulation conduit (3).

5. The medical viewing system according to any of the preceding claims, wherein the joint comprises an elastically deformable member (90) providing an elastic deformation force when moving the tubular element (20) from the unexpanded position to the expanded position, and wherein the image capturing end (8) engages the viewing cap (7) and urges the tubular element (20) towards the expanded position against the deformation force.

6. The medical viewing system according to any of the preceding claims, wherein the joint comprises a plastic deformable member providing a deformation force when moving the tubular element (20) from the unexpanded position to the expanded position, and wherein the image capturing end (8) engages the viewing cap (7) and urges the tubular element (20) towards the expanded position against the deformation force.

7. The medical viewing system according to any of the preceding claims, wherein the locking piece (21) comprises an inner tubular wall encircling a locking section of the encapsulation conduit (3), wherein the tubular element (20) comprises an outer tubular wall (93) encircling the locking section of the encapsulation conduit (3), wherein the inner wall has an inner surface towards the encapsulation conduit (3) and an outer surface towards the outer tubular wall (93), and wherein the outer tubular wall (93) has an inner surface sliding against the outer surface of the inner tubular wall during the displacement from the unexpanded position to the expanded position.

8. The medical viewing system according to claim 7, wherein the locking piece (21) comprises an external tubular wall encircling the locking section of the encapsulation conduit (3) and comprises an inner surface towards the outer tubular wall (93) and an outer surface forming a handpiece for gripping the encapsulation.

9. The medical viewing system according to claims 7 or 8, wherein the inner surface of the inner tubular wall towards the encapsulation conduit (3) defines the scope lock (30).

10. The medical viewing system according to claim 5 and any of the claims 7-9, wherein the elastically deformable member (90) is arranged in a space between the external tubular wall and outer tubular wall (93).

11. The medical viewing system according to claim 10, wherein the spring is limited in the distal end (5) of the space by the tubular element (20) and limited in proximal end (4) of the space by the locking piece (21).

12. The medical viewing system according to any of the preceding claims, wherein the tubular element (20) defines a first section of the encapsulation conduit (3) towards the distal end (5), wherein the first section of the encapsulation conduit (3) has a uniform cross section.

13. The medical viewing system according to any of the preceding claims, wherein the tubular element (20) defines a second section of the encapsulation conduit (3) towards the proximal end (4), wherein the second section of the encapsulation conduit (3) has a non-uniform cross section.

14. The medical viewing system according to any of the preceding claims, wherein the viewing scope (2) comprises a handle part from which the elongated insertion part projects, and wherein the encapsulation comprises a pouch connected to the locking piece (21) and configured for encapsulation of the handle part.

15. The medical viewing system according to claim 14, wherein the pouch is connected to the locking piece (21) in a releasable connection structure.
